# EUROPEAN PATENT SPECIFICATION

(11) **EP 4 192 630 B1**
(45) Date of publication and mention of the grant of the patent: **01.10.2025**
(21) Application number: 21852302.5
(22) Date of filing: 02.08.2021
(51) Int. Cl.: B08B 3/02, A47F 10/04, B60S 3/00, A61L 2/07, A61L 2/18

(54) **DISINFECTION ARRANGEMENT AND METHOD FOR DISINFECTING**
DESINFEKTIONSANORDNUNG UND VERFAHREN ZUR DESINFEKTION
AGENCEMENT DE DÉSINFECTION ET PROCÉDÉ DE DÉSINFECTION

(30) Priority: 05.08.2020 SE 2050936
(43) Date of publication of application: 14.06.2023
(73) Proprietor: SanArK Systems AB, 685 34 Torsby (SE)
(72) Inventor: LARSSON, Bengt-Åke, 685 34 TORSBY (SE); FOTH, Istvan, 680 51 STÖLLET (SE); STEEN, Johnny, 680 60 SYSSLEBÄCK (SE)
(74) Representative: Bjerkén Hynell KB
(86) International application number: PCT/SE2021/050765
(87) International publication number: WO 2022/031204

(56) References cited:
- EP-A1- 2 724 791
- EP-A2- 0 569 885
- EP-A2- 3 546 076
- EP-B1- 0 569 885
- DE-A1- 102007 009 805
- DE-A1- 3 828 035
- NL-B1- 2 015 723
- NL-C2- 1 007 094
- US-A1- 2008 210 268
- US-A1- 2008 210 268
- US-B1- 6 244 278
- US-B1- 6 244 278

## Description

### TECHINICAL FIELD

This invention relates to a disinfection arrangement for disinfecting a movable object, preferably adapted for shopping carts, comprising a disinfectant supply arrangement, an object inlet part, a disinfection unit and an object outlet part, wherein said disinfection unit comprises an outer cover and an inner disinfection chamber, wherein one of said disinfection chamber and/or outer cover is rotated by a drive unit that is controlled by a control unit.

### BACKGROUND

There exist many situations where virus and bacteria may be transferred from one person to another, due the ability of virus and bacteria to stay alive for a relative long time period when placed on a surface. Accordingly, there exist many items that potentially may cause spread of a disease, due to being touched by different persons during the time period when virus and bacteria may still be active. An example of such items is shopping carts and shopping baskets for use within a shop.

A plurality of different solutions is known for disinfecting such items between use of different persons. None of these solutions provide an arrangement that may meet demands required for being accepted commercially, e.g. due to complexity, cost and/or logistic deficits.

From US6244278 there is known a disinfection arrangement having a round turntable that is journaled in the bottom of a cleaning chamber, on which turntable a shopping carts to be cleaned is placed, and which during the cleaning process is made to rotate, wherein nozzles emit a cleaner on the catering carts during rotation of the turntable. For subsequent drying the rotational speed of the turntable is increased, such that the liquid cleaner is spun away from the surfaces of the carts. During cleaning and drying the catering carts are placed within a cage that is vertically slidable within the housing of the disinfection arrangement.

From EP1918030 there is known an apparatus for washing trolleyed objects provided with wheels. The apparatus comprises a washing chamber and guides on which the wheels of the trolleyed objects are able to be disposed and moved by chain movement members to selectively insert and extract a trolleyed object into and out from the washing chamber, respectively.

DE102007009805 discloses a disinfection arrangement, comprising a conveying path for vehicles, which is running in curved manner, wherein a run-out zone extends parallel to the run-in zone. The vehicles are guided into the run-in zone where washing is performed running in a first direction and guided through a turning zone to thereafter be dried in the run-out zone running in a second direction, opposite to the running in a first direction.

From NL2015723 there is known a mobile cleaning arrangement which may be used to clean nested shopping carts batch wise, comprising a cleaning chamber having an open frame which is rotatable about a horizontal axis, to achieve that the devices can be brought into contact with cleaning liquid from a greater number of angles.

US2008210268 discloses a shopping cart (and basket) washer and sanitizer having an enclosure fitted with a tunnel and components that facilitate the entry and exit of rolling shopping carts. Within the tunnel, the carts are washed, rinsed, disinfected and dried.

From WO2011076476 there is known a cleaning device for disinfecting shopping trolley handles, comprising a disinfection chamber, which can be moved such that at least one part of the shopping trolley handle can enter into the disinfection chamber and can exit from said disinfection chamber in order to disinfect at least part of the handle.

There exist disadvantages with known arrangements, e.g. from a logistic perspective and/or commercial perspective (e.g. too complex/expensive), which seems to be reasons that there exist very few disinfection arrangements, despite an evident need.

### SUMMARY OF THE INVENTION

It is an object of the invention to eliminate or at least reduce at least some of the above-mentioned problems/ disadvantages, which is achieved in accordance with a solution as defined in claim 1.

Thanks to the invention very efficient disinfection arrangement is achieved. A great advantage with the arrangement according to the invention is that the movable object that is to be disinfected may be introduced into disinfection arrangement in a convenient manner by easily pushing the handle thereof and that it after disinfection will be provided with the handle protruding outwards and therefore will be easy to grab and move away by a user. As a consequence it brings about the advantage that no one else but the user needs to touch the handle during use of the movable object, since one and the same person may first easily grab/fetch a chosen object from the disinfection arrangement, use it and and after use return it to the disinfection arrangement, without any one else touching it.

A further advantage is that the arrangement may provide for a very efficient disinfection, also by use of steam, thanks to providing an arrangement that has relatively limited space that needs to be supplied with the disinfectant. Moreover, the arrangement provides the advantage that supply and/or drive members may be provided from below which makes it easier to provide for a cost efficient design. Further, the arrangement may provide the advantage that supply of disinfectant into the disinfection chamber is achieved from a non-rotating part.

Further aspects and details of the invention will become apparent from the following detailed description.

### BRIEF DESCRIPTION OF THE DRAWINGS

In the following the invention will be described in more detail with reference to the enclosed figures, wherein;
Figure 1 shows a perspective front view of a first embodiment of a disinfection arrangement according to the invention,
Figure 2 shows a perspective view from above of the first embodiment according to the invention, but without walls and roof,
Figure 3 shows a view from above of Fig. 2,
Figure 4 shows a perspective view focusing on the disinfection unit, wherein the disinfection chamber is positioned for receiving a movable object for disinfection,
Figure 5 shows the same perspective view as in Fig. 4 but without one of the wall parts of the outer cover, wherein the inner disinfection chamber has been rotated 90 degrees,
Figure 6 shows the same perspective view as in Fig. 4 but without one of the wall parts of the outer cover, wherein the inner disinfection chamber has been rotated 180 degrees,
Figure 7 shows a perspective view from another angle of the disinfection unit, without any of the wall parts of the outer cover, wherein the drive motor is shown, and the disinfection chamber is positioned for receiving a movable object for disinfection,
Figure 8 shows a perspective view from above of the disinfection unit, without any of the wall parts and without the upper part of the bottom part, to show details of the drive for rotation of the disinfection chamber,
Figure 9 shows a perspective view from below of the rotatable bottom part of the disinfection chamber,
Figs 10 and 11 show a second embodiment of how to arrange for supply of disinfectant,
Figs 12 and 13 show an embodiment of the disinfection arrangement, wherein a plurality of parallel paths are used, and
Figs. 14 and 15 show an alternate embodiment of how to arrange for supply of disinfectant,
In Fig. 16 there is shown a perspective view without outer walls of a disinfection arrangement according to a preferred embodiment of the invention,
Fig. 17 shows a perspective view of the disinfection unit, but without the door arrangements,
Fig. 18 shows a similar view as Fig. 17, wherein the door arrangement is shown and wherein the disinfection chamber is in a position where the door arrangement seals the disinfection chamber,
Fig. 19 shows a perspective view of parts of the disinfection unit with a focus on an actuation arrangement for movement of the door arrangement between an open and a closed position, and,
Fig. 20 shows a perspective view of the disinfection unit, wherein the disinfection chamber and outer cover have been removed.

### DETAILED DESCRIPTION

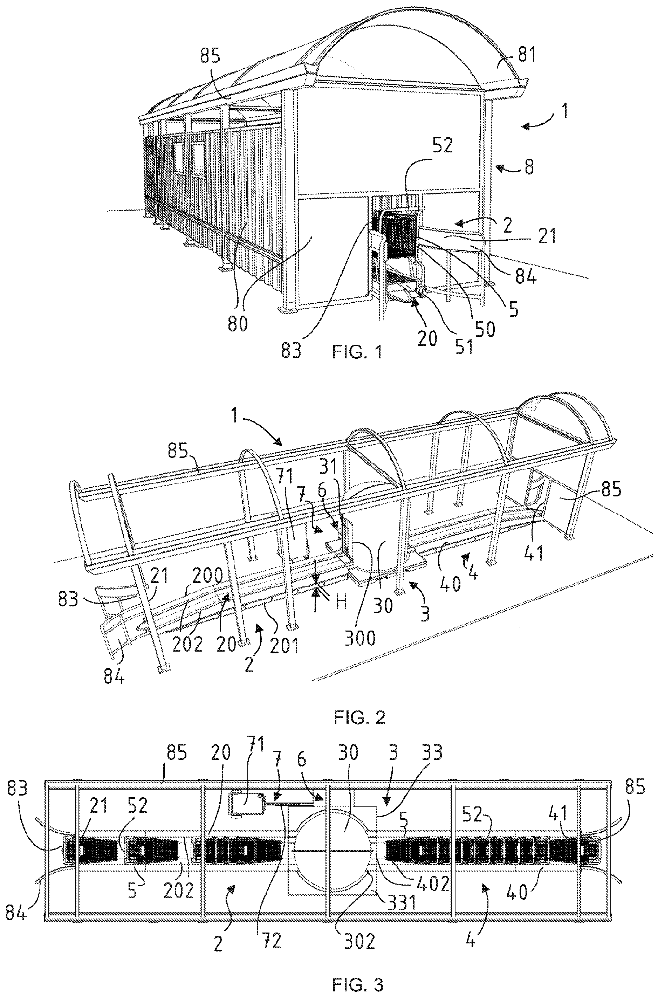

In Fig. 1 there is shown a perspective view of a disinfection arrangement 1 according to the invention seen from a front side, comprising a protecting building structure 8 having walls 80 and a roof 81 and also openings 83, 85 for inlet and outlet of movable objects 5, which here are exemplified in form of shopping carts. The shopping cart 5 includes a basic structure 50, wheels 51 and handle 52. As is shown in the figure, the shopping cart 5 is moved into the disinfection arrangement 1 through the opening 83 in the building which also forms the inlet 21 of the inlet part 2.

In Fig. 2 there is shown the same disinfection arrangement 1 in a view seen from above without walls and roof. It is shown that the disinfection installation comprises an inlet part 2 and an outlet part 4. In-between, centrally of the inlet and outlet parts there is arranged a disinfection unit 3. Within the disinfection unit 3 the shopping carts will be disinfected, (described more in detail below) and simultaneously be turned 180 degrees such that the handle 52 of the shopping cart will protrude out from the outlet 40 of the outlet part 4.

Further, it is shown that there is positioned a disinfection arrangement 6 for supply of disinfectant to the disinfection unit 3. Both the inlet part 2 and the outlet part 4 includes relatively long inlet and outlet paths 20, 40 respectively, in order to be able to provide buffer zones for a plurality of shopping carts 5. Within the paths 20, 40 there are arranged guide tracks 202, 402 arranged for housing/guiding the wheels 51 of the shopping carts 5 to securely guide the shopping carts from inlet 21 to outlet 41during the transport within the disinfection arrangement 1.

Further there is shown that the disinfection unit 3 comprises an outer cover 30 and a disinfection chamber 31 supported by a bottom part 33. In the shown embodiment the outer cover 30 is separated from the disinfection chamber 31 and provide with an inlet opening 300 and an outlet opening 302. Moreover, there is indicated a control unit 6 which includes a processor and software that automatically may control the functioning of the disinfection unit 3. There is also indicated a disinfection supply arrangement 7 including a disinfection supply device 71. There is a tubing 72, from the disinfection supply device 71 to a bottom part 33 of the disinfection device 3, which tubing 72 may first be connected to a supply control device (not shown, e.g. within the same housing as the control unit,) prior to entering into the bottom part 33. The disinfectant is supplied into the disinfection unit 3 from below via the bottom part 33 that the fixed cover 30 and rotatable chamber 31 are placed upon. The outer cover 30 is fixed onto the bottom part 33 whereas the inner disinfection chamber 31 is rotatably arranged thereon. To achieve the advantage described above, i.e. having the handle of the object 5 protruding at the outlet 40, there is a use of having two openings 300, 302 in the outer cover 30, inlet 300 and outlet 302, whereas it is sufficient to have one opening 310 in the rotatable chamber 31. If two inlet openings and two outlet openings (not shown) are used in the outer cover 30, it may be an advantage to use two openings (not shown) in the inner disinfection chamber 31, as explained more in detail at the end of the description.

Preferably each path 20, 40 is built up of easily inter fitted modules 200, 201. Preferably at least one 202 of these modules, i.e. the one positioned close to the inlet 21 includes an inclination to arrange for moving the movable objects 5 up to a higher level H. The higher level H is an advantage since it will then be easy to provide space in the bottom part 33 for the tubing and other needed equipment, i.e. to arrange for supply of disinfection and also arrange for the rotatable function of the inner disinfection chamber 31. In between the first module 200 (including the inclination) and the bottom part 33 there may be arranged a plurality of horizontal modules 201 having the same height H as the bottom plate. There are guide tracks 202 and 402 arranged within each of the modules and also within the bottom part 33 to provide for guiding the movable objects 5 from the inlet 21 to the outlet 41.

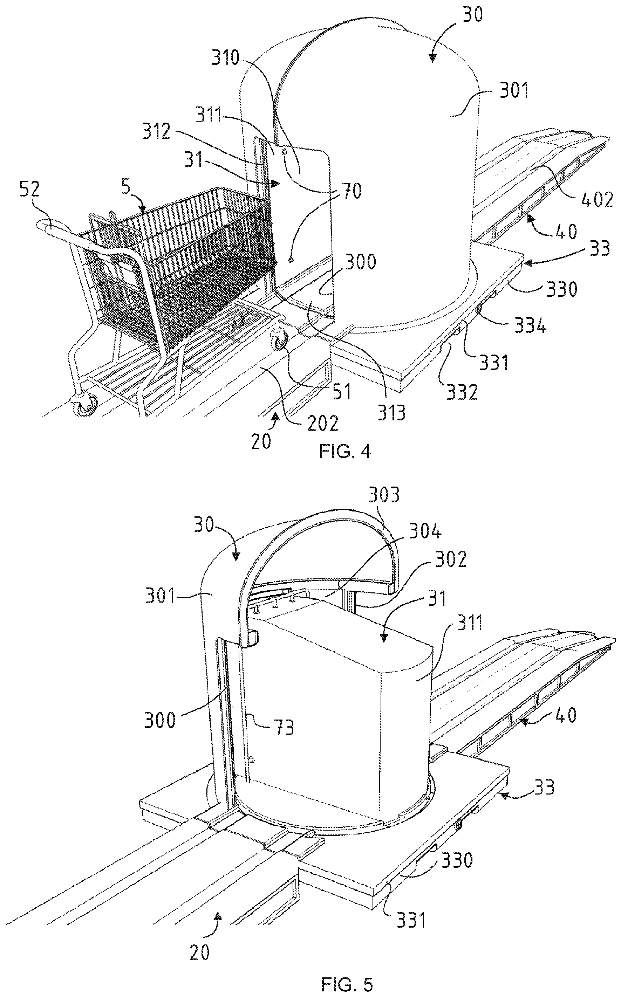

In Fig. 4 there is shown a perspective view from above wherein a movable object 5 in the form of a shopping cart is on its way to be moved into the inner of the inner disinfection chamber 31. The shopping cart 5 is having its wheels 51 within the guide tracks 202 and positioned such that the handle 52 will lastly move into the inlets 300, 310 of the inner disinfection chamber 31 and outer cover 30, respectively. The outer cover 30 is also arranged with an outlet 302, positioned on the opposite side of the outer cover 30.

The outer cover 30 has walls 301 made in a non-permeable material such that disinfectant may not penetrate through to the outside. Also, the inner disinfection chamber 31 has walls 310 that are non-permeable such that the disinfectant may not permeate through to the outside from the inner space of the inner disinfection chamber 31. Furthermore, the inner surface 304 of the outer cover 30 may be rotationally symmetric, at least partly, and around the inlet opening 310 of the inner disinfection chamber 31 there may be arranged a sealing 312 which may seal against the inner wall 304 of the outer cover 30, at least at the rotation symmetric wall parts, such that when the inner disinfection chamber 31 is rotated a sufficient angle the sealing 312 will totally seal off the inner space within the inner disinfection chamber 31.

The disinfection unit 3 comprises a bottom part 33 positioned on top of which the outer cover 30 and the inner disinfection chamber are safely positioned. The bottom part 33 comprises a lower bottom part 330 and an upper bottom part 331. Further, there are preferably passages 332 for enabling a forklift or similar to lift and move the whole disinfection unit away and back to its user position. Centrally arranged within the upper bottom plate 331 there is a rotatable inner bottom plate 313 that forms the bottom of the inner disinfection chamber 31. Preferably the outer cover 30 is made from two halves and joined via rim 303 along a dividing surface that extends in a vertical plane, such that the two halves form symmetrical halves.

In Fig. 5 it is shown a similar perspective view as in Fig. 4, but where one of the halves of the fixed cover 30 has been removed. It is shown that the inner disinfection chamber 31 has been rotated 90 degrees, which is a suitable position for performing the disinfection process, since the opening 310 of the inner disinfection chamber 31 may then be safely sealed off against the inner wall 304 of the outer cover 30. For supply of disinfectant there is arranged an upwardly extending tubing 73 that extends outside of the inner disinfection chamber 31, preferably positioned substantially in a vertical plane adjacent to the inlet 310, where the handle of the moving object 5 is positioned. The tubing extends all the way around the wall parts 301 of the inner disinfection chamber 31 and presents a plurality of branch tubes each connected to a nozzle 70, which will spray the disinfectant into the disinfection chamber 31. Preferably the disinfectant is hot steam and thanks to sealing off the inner disinfection chamber 31 and also having a snug fit in relation to the moving object 5 the space therein will be relatively limited and therefore quickly and efficiently heated to disinfecting temperatures (e.g. above 70 C°) by means of the steam that reliably will disinfect the movable object 5 and especially around parts that include the handle 52. As shown in Fig 5. the inner disinfection chamber 31 may merely be arranged with one opening 310, enabling an adapted design of the walls 311 to optimize (e.g. minimize) the space within the disinfection chamber 31.

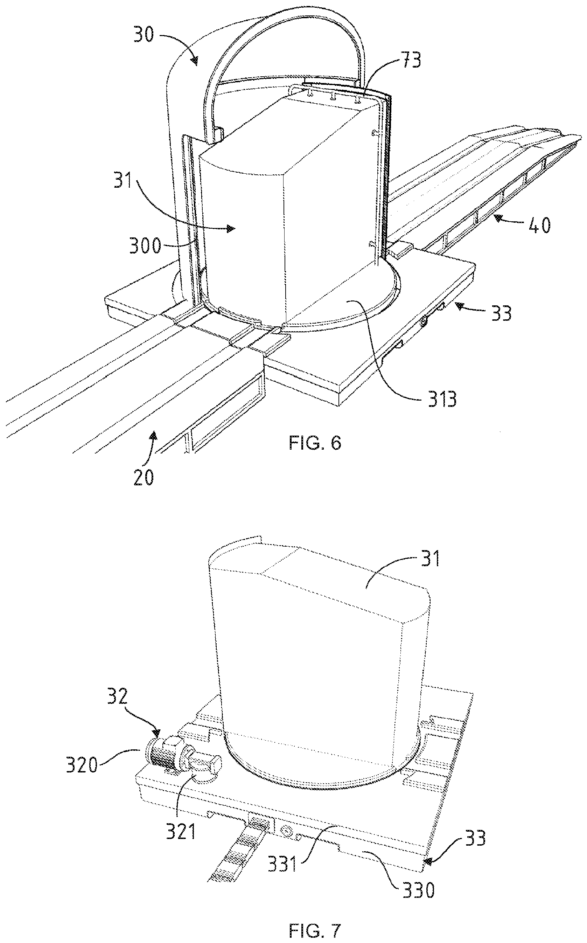

In Fig. 6 there is shown a position wherein the inner disinfection chamber 31 has rotated further 90 degrees and accordingly now having its opening 310 in line with the outlet 302 of the outer cover. Hence, the movable object 5 may now be moved out from the disinfection unit 3 and onto the outlet path 40. Once the movable object 5 has been taken out from the disinfection arrangement 3 the inner disinfection chamber 31 will again be rotated such that its inner opening 310 will be in line with the inlet opening 301 of the outer cover 30.

In Fig. 7 there is shown a perspective view from above wherein the outer cover 30 has been totally removed and only the inner disinfection chamber 31 is shown. There is shown that a drive unit 32 is attached to the bottom part 33 to provide for rotation of the inner disinfection chamber 31. The drive unit 32 comprises a motor 320 and gear transmission 321, preferably in form of a gear mechanism 321 (e.g. worm wheel) that will transfer rotation from a horizontally positioned shaft of the motor 320 to a vertically positioned shaft including a first drive member 322.

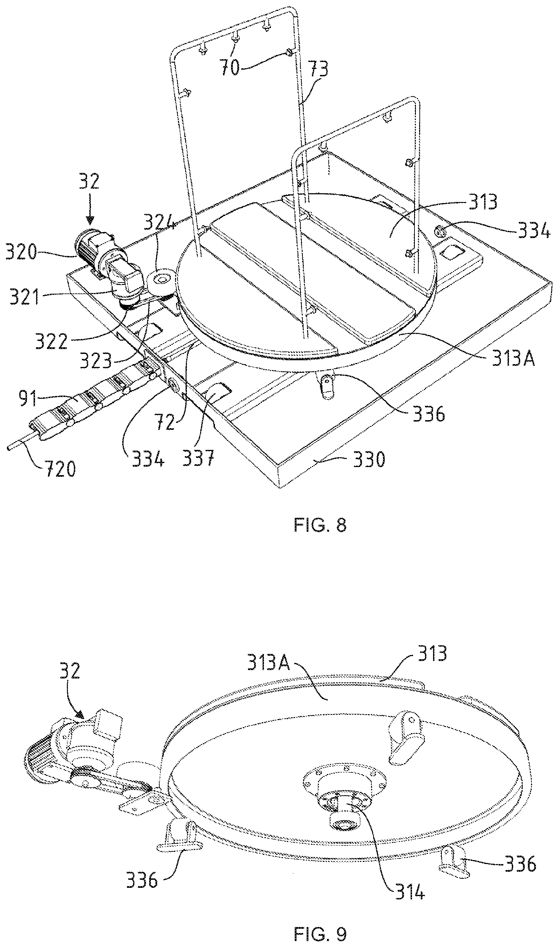

In figure 8 merely the lower part 330 of the bottom part 33 is shown in the figure, such that parts that are positioned underneath the top surface of the upper bottom part 331 are also shown. As shown in Fig 8, the first drive member 322 may transfers rotation to a second drive member 324, preferably via a belt 323. The second drive member 324 may transfer rotation onto the rotatable inner bottom plate 313 of the inner disinfection chamber 31. In a preferred embodiment the second drive member 324 is in the form of a friction wheel 324 that in contact with the outer peripheral surface 313A of the rotatable inner bottom plate 313 will transfer rotation to the inner disinfection chamber 31. The inner rotatable bottom plate 313 is positioned on friction reducing members 336 which will provide for a substantially friction less support of the rotatable bottom 313. In a preferred embodiment the friction reducing members 336 includes wheels that support directly onto the bottom surface of the rotatable inner bottom plate 313.

Furthermore, it is shown that the lower bottom part 330 preferably may be provided with roller wheels 337 that may be used to provide for a movability of the whole bottom part 33, which is a great advantage if one and the same disinfection unit 30, 31 shall act as single disinfection unit for a plurality of paths, as will be explained more in detail below. It is also shown that according to the preferable embodiment there are arranged attachment means 334 for attaching a drive member, e.g. cable, and further a flexible supply member 91 (protecting a flexible supply tube 720) for providing possibility of movement of the disinfection unit 3 (as will be explained more in detail below). However, it is evident for the skilled person that a fixed disinfection unit may be provided with a bottom without details enabling movability.

In Fig. 9 there is schematically shown a view from below of a first embodiment of the bottom plate 313 of the inner disinfection chamber 31. It can be noted that the bottom part 313 has a centrally arranged stub shaft 314. This stub shaft 314 may fulfil several functions. Firstly, it may fulfil the function of keeping the inner disinfection chamber 31 in a position that safeguards rotation around a fixed axis within the bottom part 33. Furthermore, it may be arranged in a form of hollow stub shaft 314 providing passage for disinfectant from the supply unit 71 via tubing 72 connected to said stub shaft 314, e.g. by means of a rotatable coupling (not shown) and further via branch tubes that are arranged in the bottom plate 313 (not shown), to supply disinfectant to the tubing 73 around the inner disinfection chamber 31.

It is foreseen that the arrangement according to the invention may be used for supply of a variety of disinfectant and also that it may be used for disinfection of a plurality of movable objects, having handles or not, despite the fact that the invention is especially suitable for disinfecting movable objects provided with handles. Accordingly, it is foreseen that the invention, and the basic functions thereof, may be used in other applications than for disinfection of items used for shopping. Moreover, it may also be used without actual disinfection, e.g. for cleaning movable objects.

During use of the disinfection arrangement 1 according to the invention movable objects 5 are supplied to the inlet opening 83, wherein a drive arrangement (not shown) will arrange for transfer onto the inlet path, wherein a plurality of movable objects 5 may be stacked/stored to provide a buffer. The movable object 5 closest to the disinfection unit 3 will (by means of the same or a second drive arrangement, not shown) be moved into the inner disinfection chamber 31once this is in position (see Fig. 4). Thereafter, the drive unit 32 will rotate the inner disinfection chamber 31 and disinfection will be achieved, as described above. The inner disinfection chamber 31 is the again rotated back to its receiving position, for repeated operation. The operation is preferably totally automatically controlled by the control unit 6, without need of any himan support.

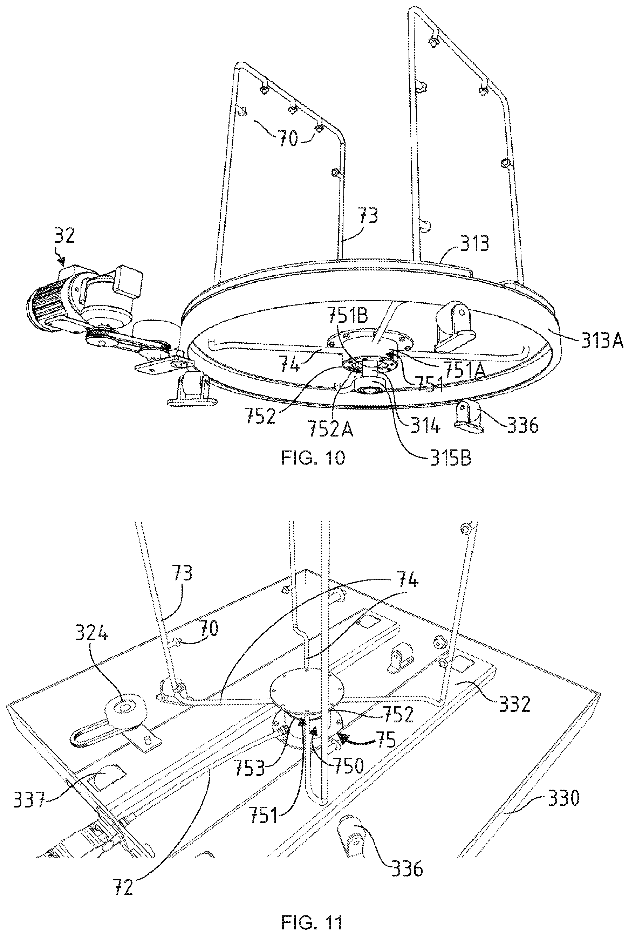

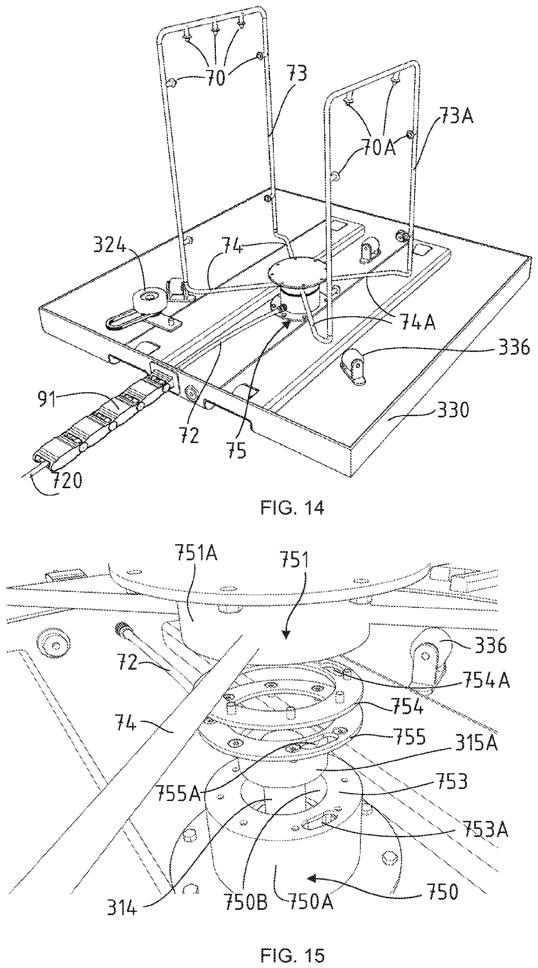

In Figs. 10 and 11 there is shown a further embodiment for supply of the disinfectant. In this embodiment, the disinfectant, preferably in the form of steam is supplied to a hub 75 that will transfer the steam further to supply tubes 74 fixed to the inner bottom 313 and further to the supply tubes 73 fixed to the inner disinfection chamber 31. The supply tubing 72 may include an outer portion 720 including a flexible hose (see Fig. 8) embedded in a flexible transfer chain 91. Further, there is a fixed supply tubing 72 arranged in the lower bottom part 330, which is connected to a lower half 750 of the supply hub 75. Further, there is an upper half 751 of the hub 75 that is fixed to the rotatable inner bottom member 313. Accordingly, the upper half 751 will follow the rotation of the inner disinfection chamber 31. Each half 750, 751 comprises an outer cylinder 750A, 751A and an inner coaxially arranged cylinder 750B, 751B, each pair forming an annular space. An upper ring 752 substantially seals the upper half 751 and a lower ring 753 (see Fig. 15) substantially seals the lower half 750. Hence, the rings 752, 753 sealingly divides the upper half 751 from the lower half 750. Both rings are arranged with an oblong passage 752A, 753A that when overlapping will allow communication between the two halves 750, 751, e.g. steam to pass from the lower half 750 to the upper half 751. The stub shaft 314 is provided with a lower 315B and an upper bearing 315A (see Fig. 15) fitting within the inner hollow space, i.e. in contact with the inner cylinders 750B, 751B, to provide for frictionless fixitaion of the rotation centre of the rotatable bottom 313.

Thanks to this arrangement the supply of steam will occur at a predetermined angle range, i.e. when the two oblong passages 752A, 753A are overlapping. **In** a preferred embodiment this is arranged for when the inner disinfection chamber 31 has its opening 310 sealed against the inner wall 304 of the outer cover 30, e.g. during a range of 10-45 ° of the 180° rotation of the inner disinfection chamber 31. **In** one embodiment the inner disinfection chamber 31 will rotate at a preset/determined constant speed that is adapted to a desired amount of steam supply during passage of the oblong passages. **In** another alternative, rotation may be performed stepwise and moved more quickly to a position where steam is being supplied and then further once sufficient steam supply has been provided. It is evident that this embodiment may function without any further valves, but as is evident for the skilled person there may of course also be both manually operated and/or automatically operated valves connected to the supply to obtain for further controllability of steam supply, or other desired fluid/s.

Preferably there are sensors within the inner space of the inner disinfection chamber 31 that will measure certain parameters, e.g. temperature, to safeguard sufficient supply to arrange for secure disinfection.

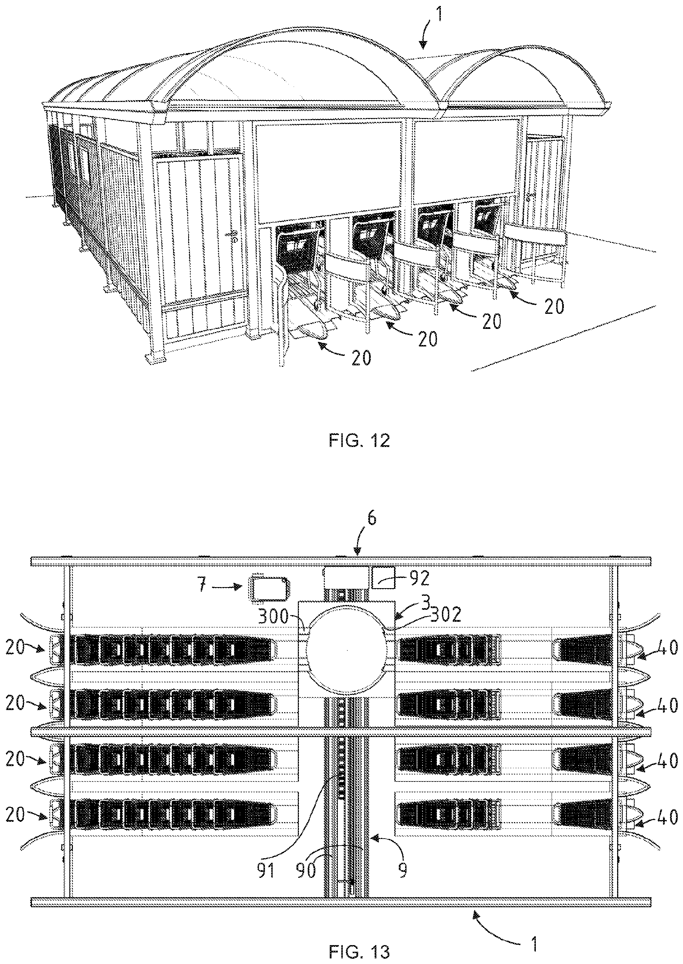

**In** Figs. 12 and 13 there is shown a further embodiment according to the invention. **In** this embodiment a plurality of paths 20, 40 are arranged within the disinfection arrangement 1. The principle how each path 20, 40 is operated is the same as has been described above.

**In** Fig. 13 it is presented that merely one disinfection unit 3 is provided for assisting all of a plurality of paths 20, 40, by means of being movably arranged transversally in relation to the paths 20, 40. A transversal track 9 may be arranged within the disinfection arrangement 1 having guide tracks 90 along which the wheels 337 at the bottom of the bottom part 33 may be guided. A drive member 92 is arranged for moving the disinfection unit 3 along the track 9. Further, there is a flexible supply member 91 including the flexible tubing 720 for supply of the disinfectant. Accordingly, the disinfection arrangement may be moved transversally in relation to the paths 20, 40 and assist with disinfection of movable objects 5 within each of the paths. The control unit 6 may preferably be connected to sensor devices (not shown) that can identify which of the paths 20, 40 that has the most movable objects 5 stacked therein, such that a prioritization may be obtained for optimizing the process.

**In** Fig. 14 there is shown a further alternate embodiment according to the invention where there are arranged two fixed vertically extending tubings 73, 73A to provide for a more powerful steam supply or other disinfection supply, thereby enabling further nozzles 70, 70A for supply of disinfectant. Thanks to using a hub 75, a plurality of branch tubing may easily be connected to the upper part 751 of the hub 75 for dispersed supply of the disinfectant.

**In** Fig. 15 there is shown a slightly alternate embodiment according to the invention of the hub 75. It is shown that in-between the lower half 750 and the upper half 751 of the hub 75 there is a sealing arrangement, that also provides for minimized friction in the form of two sealing rings 754, 755 fitted to the upper half 751 and lower half 750, respectively. Both sealing rings 754, 755 are arranged with the same kind of oblong passages 754A, 755A as those passages 752A, 753A of the fixed rings 752, 753. The function is the same as described above, i.e. when overlapping steam may pass from the lower half 750 to the upper half 751.

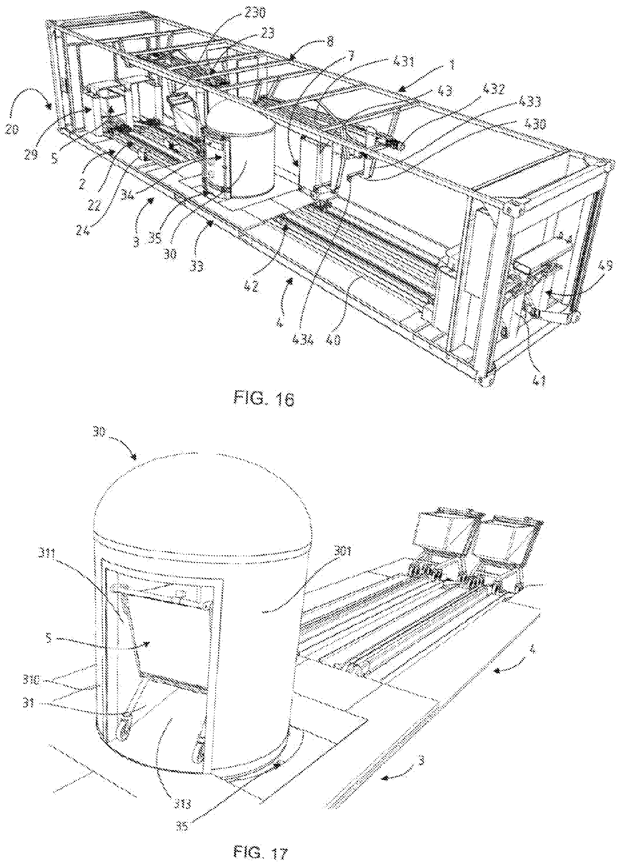

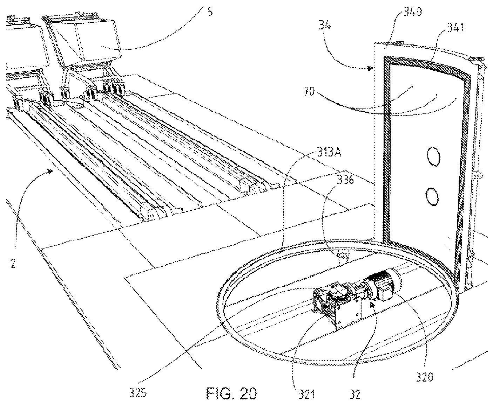

Figs. 16-20 show some preferred details of a disinfection arrangement according to a preferred embodiment of the invention. In the following there will be focus on specific details that are present regarding the preferred embodiment. Basically, many features of the preferred embodiment are the same as for the above described embodiments, as is evident from the reference numbers used and there will therefore not be repetition of such basic details.

In Fig. 16 there is shown a perspective view of a disinfection arrangement 1 according to the invention, wherein the inlet part 2 is at a distal end thereof and the outlet part 4 is at the closed end of the disinfection arrangement. The disinfection arrangement 1 is shown with the framework of the protecting building structure 8 but without any outer walls or roof. Starting at the inlet part 2 there is shown one novel aspect that is not shown in any of the other embodiments, namely a gate member 29 at the inlet opening 21 of each inlet path 20, i.e. here two. Each inlet gate members 29 includes locking members (not shown) that will enable locking of a movable object 5 within the inlet opening 21 such that when the locking member is activated it will not be possible for someone to enter into the disinfection arrangement 1, since the movable object 5 will block the inlet and thereby hinder passage into the disinfection arrangement 1.

Further, it is shown that there are two drive arrangements 22, 23, for movement of an object 5 from the inlet 21 into the disinfection unit 3. There is a first drive arrangement 22 moving the movable object 5 from the inlet 21 to a position nearby the disinfection unit 3 and a second drive arrangement 23 arranged for moving the object 5 from an inner position into the disinfection unit 3. In the preferred embodiment the first drive arrangement 2 is positioned along each path 20 within or on the floor of the disinfection arrangement 1 and the second drive arrangement arranged adjacent the ceiling of the disinfection unit arrangement 1. Accordingly, the first drive arrangement 22 will be equipped with holding/pushing members that protrude upwardly to get in contact with the object 5, whereas the second drive arrangement 23 will be arranged with downwardly protruding gripping members 230, 430 to have gripping contact with the movable object 5.

The second drive arrangements 23, 43 have downwardly protruding gripping members 230, 430 supported and guided by guiding devices attached above the objects 5. Preferably guiding devices comprises rails 431. More preferred it comprises a plurality of shaped rails 431 that may guide the carts 5 into and away from one disinfection unit 3 from and onto a plurality of guide paths 202, 402, along the inlet 202 and outlet paths 402, respectively. Each downwardly protruding gripping member 430 include an L-shaped body portion 433 to provide a horizontally extending end part that may be moved into the disinfection space 313. At the horizontally extending end part there is arranged some kind of gripping device 434.

Further, it is shown that the outer cover 30 is rotatable together with the disinfection chamber 31. Preferably by being attached to each other. A door arrangement 34, 35 is arranged at a fixed position in relation to the rotatable disinfection chamber 31 and cover 35. The door arrangement 34, 35 comprises a door member 34 that fits onto the opening 301 of the disinfection chamber to arrange for sealing of the inner space 310 of the disinfection chamber 31. An actuating arrangement 35 may move the door member 34 from an open position to a closed position where it preferably will seal the disinfection chamber 31, such that disinfection may efficiently be performed within the inner space 310 of the disinfection chamber 31.

Further, it is shown that the outlet part 4 is mirror symmetric in regard to the inlet part 2. Accordingly, also the outlet part has two parallel paths 40 and two drive arrangements 42, 43 for movement of the objects 5. Also, at the outlet 41 of each path 40 there is arranged a gate member 49 that may lock an object 5 within the gate member 49 to eliminate the possibility to enter into the disinfection arrangement from the outlet 41.

In Fig. 17 there is shown a perspective view wherein the door arrangement 34, 35 of the disinfection unit 3 has been removed. It shows the joint inlet opening 310 of the disinfection chamber 31 and the outer cover 30 and that there is an object 5 positioned within the disinfection chamber 31. It is shown that preferably the disinfection chamber 31 is arranged with walls 311 that are adapted to the form of the object 5 to minimize the total space 313 within the disinfection chamber 31 to be able to more efficiently perform disinfection of an object 5. In the shown view the disinfection chamber 31 and the outer cover 30 are positioned in an inlet position, wherein the opening 310 is in line with the inlet path 20, to enable introduction of an object into the inner space 313 of the disinfection chamber. In the figure there is also indicated an actuating member of the actuation arrangement 35, for movement of the door member 34 (not shown).

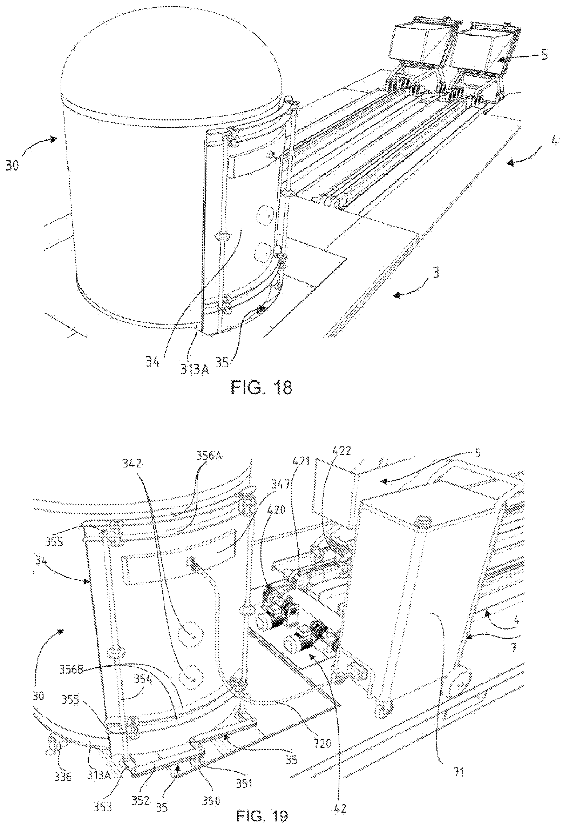

In Fig. 18 there is shown a similar perspective view as shown in Fig. 17, wherein the disinfection chamber and the outer cover 31, 30 have been rotated 90°. In this figure the door arrangement 34, 35 is also shown and at a position, wherein the actuation arrangement 35 has moved the door member 34 into a closed position where it seals the opening 310 of the disinfection chamber 31, such that efficient disinfection may be performed preferably by the use of hot steam.

In Fig. 19 there is shown a more detailed view of the disinfection unit 3 and the beginning of the outlet part 4, wherein the top cover 330 of the bottom part 33 of the disinfection unit 3 has been removed. As a consequence, the actuating arrangement 35 of the door arrangement 34, 35 is shown in more detail. There is shown that the actuation arrangement 35 includes a rotatable shaft 350 that is actuated by a motor (not shown). Connected to the rotatable shaft there is a T-shaped member connected to levers 352. Each lever 352 is in turn connected to a lever arm 353 fixedly attached to vertically positioned rotatable rods 354. Each rotatable rod 354 is in turn connected to an upper and lower pivot member 355 attached to horizontal attachment members 356A, 356B. The horizontal attachment members 356A, 356B are positioned with a relatively large distance apart such that one is positioned adjacent the upper end of the door member 4 and another one is attached adjacent the lower end of the door 34.

Accordingly, there is a rigid connection between the actuating arrangement 35 and the door member 34 for movement of the door member 34 between the closed and the open position. Movement of the door member 34 is achieved by rotating the drive shaft 350, whereby the T-shaped member 351 will push/pull the levers 35, which in turn will arrange for rotation of the vertical rods 354, such that the pivot mechanism 355 will arrange for movement of the door member 34 to and from the inlet opening 310 of the disinfection chamber 31.

Further, it is shown that in a similar manner as described above, the bottom part 313 of the rotatable disinfection chamber 31 and here also the cover 30, is arranged with a peripheral member 313A providing a downwardly exposed surface that will interact with wheel members 336 to enable rotation of the disinfection chamber and cover 30 without any noticeable friction.

Further, Fig. 19 shows that there is arranged a motor and gear arrangement 420 for activation of the first drive arrangement 42 that may move the object 5 from an intermediate position within the disinfection arrangement and closer to the outlet 41. It is shown that in a preferred embodiment the drive member 321 of the second drive arrangement 42 is in the form of a continuous loop formed drive member 241 having transport members 422 attached thereto that may move the object 5 along the path 40. A corresponding arrangement is used at the inlet part 2, and a preferred embodiment of that will be described more in detail below.

Moreover, it is shown a disinfection supply unit 71 having a supply tube 720 connected to the disinfection unit 3. In the preferred embodiment this connection is with the door member 34, such that merely a rather limited movement of the supply tube 720 is needed. The door member 34 is preferably arranged with a supply device 347 that extends along a substantial portion horizontally at the upper part of the door member 34. Within the supply device 347 there is an open space which in turn is in connection with outlet nozzles 70 for providing disinfection fluid into the inner space 313 of the disinfection chamber 31, when the door member 34 is in its closed position. Moreover, there is also shown that at least one sensor 342 may be applied in the door member 34, e.g. to sense the temperature within the disinfection chamber 31. More preferred there are two sensors 342 applied at different levels, enabling sensing in more detail and also providing a back-up if one should fail.

The transmission 321 is arranged to have an output member 325 that is positioned centrally underneath the bottom 313 to affect the rotation. Further, it is shown that the door member 34 preferably is arranged with a sealing 341 that is positioned within the inner periphery of the body 340 of the door member and which may efficiently seal around the inlet opening 310 of the disinfection chamber , such that disinfection fluid that is supplied via the supply nozzles 70 may efficiently provide disinfection within the inner space 313 of the disinfection unit.

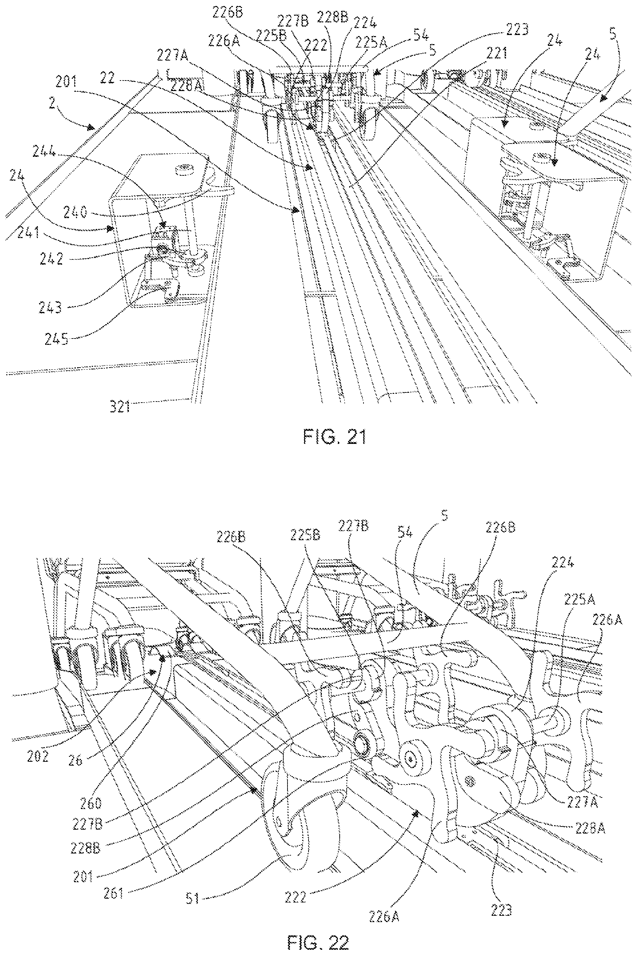

In Figs. 21-24 there are shown different perspective views of a design of a first transportation arrangement 22. Basically, the preferred first transportation arrangement 22 comprises a bringer 222 arranged at a floor level including at least one pushing device 226 that may push onto a frame member 54 of an object, e.g. cart 5, for transporting the object from a position adjacent the inlet 21 to a delivery position. The bringer 222 includes a carrier member 223 that is attached to a drive member 221, e.g. drive belt, which is driven by a drive device 229. The drive device 229, e.g. electric motor and reduction gear, is arranged to drive the drive member 221 reciprocally, forth and back between the inlet 21 and the delivery position. At the delivery position the second transportation arrangement 23 may take over and transport the cart 5 into the disinfection unit 3.

The carrier member 223 is positioned substantially at the bottom level of the inlet paths 200, 201 and may be arranged with an upwardly protruding support body 224. The support body 224 supports a pulling arrangement 225, 226 that may safely grip a frame member 54 of a cart 5 and thereby pull a cart 5 from the inlet position to the delivery position. In the preferred embodiment the pulling arrangement 225, 226 includes at least one rotatable member 226 A, B having a plurality of radially extending arm members that when positioned extending upwardly will extend above a level of the frame member 54, such that a pulling surface (facing the disinfection unit 3) may push on the frame member 54 to move the cart towards the disinfection unit 3. A locking mechanism 227, 228 in a non-activated state safeguards that the pulling member/s 226A, B can merely be rotated in one direction. In the other direction the pulling member/s 226 A, B will be blocked from rotation, if the locking mechanism 227, 228 is in non-activated. (In the shown example there is shown a plurality of pulling members 226A, B, locking mechanism members 227A,B, 228 A,B, but it is evident for the skilled person that merely one member may be used to fulfil the basic function)

Thanks to this arrangement the cart 5 may be pushed in contact with the backside of an arm of the pulling member 226A, B and pushed further on in a forward direction to thereby rotate the pulling member 226A, B such that a subsequent arm member of the pulling member 226A, B will be in an upwardly extending position, with the pulling surface in contact with frame member 54. Hence, a cart may always be pushed past a pulling member/s 226A, B but may merely be pulled backwards when the locking mechanism 227, 228 is activated releasing the pulling member/s 226A, B.

Accordingly, in a preferred embodiment the pulling member 226 A, B is freely rotatable in a forward direction but merely rotatable in a rearward direction when a locking mechanism 227, is activated to be in a release position whereby a cart may be pushed into the inlet, without hinder but not pulled out again once introduced into the inlet.

Further, in the preferred embodiment there is a delivery position mechanism 24, 24' arranged at a defined delivery position. This delivery position mechanism 24 will safeguard that a cart 5 ready for being gripped by the second transportation arrangement 23 will be positioned in an exact position for gripping. Preferably the delivery position mechanism 24, 24' includes a stop member 240 that in contact with a frame member 56 will stop and hold a cart 5 at the delivery position and that by means of a release mechanism 244, 242 may release the cart 5 from the delivery position to make it movable by the second transportation arrangement 23.

Moreover, according to a preferred embodiment a second portion 201of the inlet path path 20, along the first transportation arrangement 22, is arranged with a buffer zone wherein a plurality of carts 5 may be stored before delivery into the disinfection unit 3. Accordingly, the preferred solution includes a drive arrangement for the first transportation arrangement 22 that enables the carrier member 223 to deliver a cart into a row of a plurality of carts 5, which implies a shorter way to a position that is at a distance from the position that the carrying member will have if delivering a cart 5 to the delivery position. In order to safeguard reliable delivery of a cart 5 at the back of a row of carts there is arranged a drive control arrangement 25 that safeguards delivery at an intended position also at the back of a row of a plurality of carts 5. This is preferably based on having a drive control arrangement 25 that will stop and reverse the carrier member 223, i.e. stop it from moving further in a forward direction and also assist in making the drive mechanism 220 change direction of drive of the belt 221 to return the carrier member 223 to the inlet and its receiving position, when a predetermined level of pushing force is reached, above the force level needed to move/push a cart 5 along the path. In the preferred embodiment the drive mechanism 220 will provide for a relatively fast movement of the belt 221, that roughly may correspond to the time for a person to supply of a new cart 5 into the inlet opening normally will be facilitated. According to a preferred embodiment the speed of the carrier member 223 of the first transport arrangement is at least 0,5 m/s preferably within a range of 0,6-1,5 m/s. The length of the path 20 is preferably in the range of 2 - 10 m, including a buffer zone. Moreover, the buffer zone is preferably arranged such that at least 5 objects may be positioned within the buffer zone, preferably a number within the range of 6-20 objects 5.

Moreover, in the preferred embodiment, as has been mentioned above, there is arranged a gate member 29 having a locking device (not shown) that will securely keep a cart within the inlet position, thereby hindering anyone/thing moving into the housing. In combination with the gate member 29 the arrangement 22 for moving a cart 5 from the inlet to the delivery position is preferably arranged with a front pulling member 226A, a rear pulling member 226B. Each preferably comprising a pair of pulling members 226A, 226B. Thanks to this arrangement there may always be a cart 5 positioned within the gate member 29, also when delivering a cart 5 from the inlet position to the delivery position. Also the rear pulling member 226B will then be arranged with a release mechanism and that safeguards that a cart 5 may be pushed into the inlet with its frame member 54 into a position past the first pulling member 226B, to thereby arrange for safe positioning of a newly introduced cart 5 within the inlet in a position where it may not again be taken out from the inlet due to a locking mechanism hindering the second pulling member to allow the frame member 54 to pass backwards.

When two carts 5 are positioned in the inlet 21 the front one of them may be moved to the delivery position and allowing the last introduced cart 5 to stay in a locked position within the gate member 29. In a preferred embodiment this may be achieved by the use of a release mechanism 26 that will enable the first pulling member 226A to maintain its grip with the frame member 54 of the cart 5, whereas the release mechanism 26 releases the pulling function of the second pulling member 226B releasing it from contact with the frame member 54 of the second cart such that the carrying member 223 may be moved along the transportation path pulling only the first cart 5 to the delivery position. Preferably the release mechanism 26 includes a camming surface 260 arranged along the path that will get in contact with the end of a release device 228B which will release the locking function of the pulling member 226B to allow it to freely rotate thereby releasing the second cart 5 from the carrier member 223.

Thanks to design a plurality of carts 5 may preferably be pushed in and onto the path 20, i.e. more than only two, (if no gate lock is activated), such that more than one (e.g. two or more) cart 5 may be transported at the same time by the carrier member 223, to the delivery position. Hence, this enables flexibility, e.g. enabling the control unit to control what number of carts 5 that is being moved at the time.

In the following some features of the embodiment shown in Figs. 21-24 will be described in more detail regarding specific solutions, which otherwise may be solved by other equivalent means in accordance with the understanding of the skilled person.

In Fig. 21 there is shown that the bringer 222 comprises one carrier member 223 that in turn carries two pulling member/s 226A, B. One 226A at the front part and one 226B at the rear part. These pulling member/s 226A, B include a hub having a plurality of arms, preferably curved, protruding radially. Accordingly, in this specific embodiment the pulling member/s 226A, B are in the form of a hub portion having four curved arms protruding radially to create a gripping surface on one side and camming surface on the other side. The griping surface is formed to create a kind of recess when protruding upwards in which recess the frame member 54 of the cart 5 may be safely held/gripped, before and during being pushed into the disinfection arrangement 1. The backside of each arm will thanks to the curved form softly enable the frame member 54 of a cart to slide past it and at the same time as the pulling member/s 226A, B rotate, i.e. 90° if four arms, 120 if three arms, etc. Thanks to having four arm members on each pulling member 226A, B a rather simple design may be used to achieve that there will always be one arm member positioned protruding upwards in a position for gripping a frame member 54.

Further, Fig. 21 shows details of a preferred delivery position mechanism 24, 24'. As can be noted there is one pair of delivery position mechanisms 24 at each side of a first path and a second pair 24' for the second path. Similar holding members 240 are used also in the delivery position mechanisms 24 to hold a frame member 56 of the cart 5 at a defined delivery position, as are used for the pushing members 226. As can be noted in Fig. 21 the rear legs 56 of the cart 5 may be chosen to hold a cart 5 in the predefined position by means of the delivery position mechanisms 24. When a cart is in that position and ready to be delivered into the disinfection unit 3 by means of the second transportation arrangement 23 the gripping members of the second transportation arrangement 23 will grip onto the handles of the cart and simultaneously or slightly before the delivery position mechanisms 24 will release the blocking member 243 to enable the holding members 240 to rotate and thereby allow a cart to move further forward along the path. The delivery position mechanisms 24 preferably comprises a ratchet wheel 242 that interact with a lever arm 243. When the lever arm 243 is in its non-activated position (e.g. spring biased) it will lock the shaft 241 of holding member 240 hindering it from further rotation. When the release device 244 (preferably a solenoid) is activated it will pull the lever arm 243 such that the outer end will pivot away from being in contact with the ratchet wheel 242. At the same time the further lever mechanism 245 will arrange for synchronising the same movement at the delivery position mechanisms 24 on the other side of the path.

In 22 it is shown in more detail how an exemplary embodiment of details comprised in a bringer 222 in accordance with a preferred embodiment of the invention. It is shown the inlet part 200 and a portion of the inner part 201 of the inlet paths. It is shown that there are preferably a pair of pulling members 226A, 226B, i.e. one on each side of the support body 224, which then preferably is centrally positioned along each guide track 202, 402. Hereby it is safeguarded that balancing is achieved to avoid any clamping action when moving a cart 5. Further, as has been described above there is preferably also two such puling mechanisms A, B, each having a pair of pulling members 226A, 226B, provided onto one support body 224 to enable having one cart in a locked position within the gate 29.

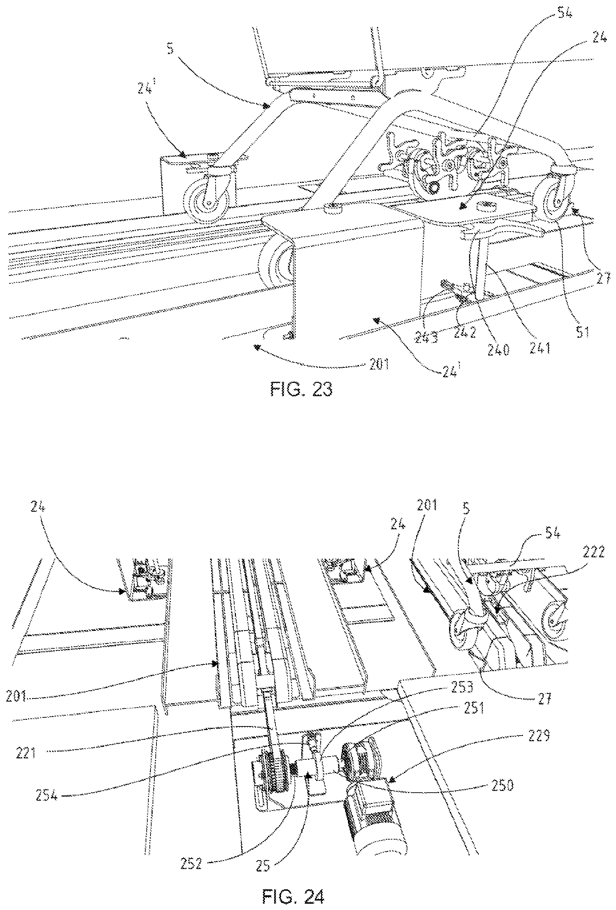

In Fig. 23 it is shown in more detail that the delivery position mechanisms 24 and 24' may preferably hinder the rear legs 56 of a cart on each path, and that they thereby may be positioned out of reach of the front legs of the cart 5.

In Fig. 24 it is shown in more detail how the drive control arrangement 25 is arranged in its exemplary embodiment, to arrange for having the carrier member 23 moving forth and back along the path to be able to build up a buffer at the inner part 201 of the path. This is achieved by having a drive control arrangement 25 that provides a stop and return function that will identify when a cart 5 has reached the end position in a row of carts 5 in a buffer. This is achieved by having a drive control arrangement 25 that my sense when the pulling/pushing resistance exceeds a certain limit, which safeguards that the pulling/pushing force of the carts in a buffer cannot exceed a certain limit. A sensor arrangement 253, 254 will sense when the pushing resistance exceeds the pre-set limit giving a signal to the control unit 6, which will actuate change of the drive direction of the drive device 229, such that the carrier member 22 will return to the inlet and be ready to fetch a new cart to be transported to the buffer.

In the shown example this is achieved by arranging a rotating output shaft 251 having splines, from the drive device 229. A socket 250 is connected with a splined end (at the inner surface) to the splined shaft 251 and may accordingly not rotate in relation to the shaft 251 but may move axially along the spline shaft 25. At the other end of the socket 250 (at the inner surface) there are arranged threads that interact with threads of a drive shaft 252 that is fixedly connected to a drum for driving the drive member 221. A spring, or similar resilient device (not shown) is arranged within the hollow space of the socket 250, e.g. between the spline shaft 251 and the socket 250 (or vice-versa), which urges the socket 250 into an end position of the threads of the drive shaft 252. i.e. in contact with a fixed stop, e.g. the drum. (or if vice-versa a stop within the socket 250) On the socket 250 there is arranged a sensor member 253 (e.g. a ring protruding radially) that interacts with a fixed optical sensor device 254. As long as the sensor member 253 is out of position, e.g. not in front of the sensor device 254 the motor 220 will drive the belt 221 in the direction towards the disinfection unit 3. When a cart 5 comes into contact with another cart directly or with the delivery position mechanism 24 the counterforce will exceed the force that normally is needed to push a cart and as a consequence the threads will start to interact, overcoming the force from the spring and will therefore start urging the socket 250 axially until the sensor device 254 senses that the sensing member 253 is in a "stop and change direction" position. When this position is reached the sensor device 254 will give a signal to the control unit 6 and the drive direction of the motor will be reversed, such that the carrier member 22 will return to the inlet and fetch a new cart to be transported to the buffer. Also, the latter may be achieved vice-versa, i.e. either stop and change when sensing the sensor member 253 or when not sensing it 253.

As is evident this will be continuously performed and controlled by the control unit 6 as long as there is more than one cart in the inlet until the buffer zone is filled to its maximum, e.g. 5-15 carts in each row. Thereafter there will normally be some kind of signal, e.g. red light at the inlet zone indicating to users that no more carts can be pushed into the inlet.

It is evident that the skilled person may find various other kind of mechanical solutions providing the same specific function as what has been described in the exemplified embodiment. Accordingly, the solution for the first transport arrangement 22 is not limited to the exact example that has been shown and described above. Furthermore, it is foreseen that a divisional application may be filed for protection of the basic principles of the first transportation arrangement 22, without any limit regarding how the cart is being moved further on into the disinfection unit and/or if the cart is taken out from the disinfection arrangement with the handle first or not. Likewise, there may also be separate application filed also for the second transportation arrangement 23 or for a combination of the two transportation arrangements 22, 23, along the same line as mentioned.

It is evident for the skilled person that the invention is not limited to what is described above but may be varied within the scope of the claims. It is evident for the skilled person that the basic principle of the invention may also work if the inner disinfection chamber 31 is arranged with two openings, since also when having two openings a sealing function may be achieved. Further it is realized that the outer cover 30 may be arranged with more than one outer inlet and outlet, e.g. two pairs of outlets and inlets, with about 90 degrees between each opening, such that sealing of two openings of the inner disinfection chamber 31may obtained in each intermediate position between each outer opening. Two separate inlet paths, 90 degrees apart, may then cross wise be activated to introduce a new object, whereby the already disinfected object may be pushed out from the disinfection unit 3 by the new object (or pulled out by a pull-out device, not shown, to avoid any contact between two objects) without need of rotating for introducing a new object. Hence, in such an arrangement there is no need to rotate more than 90 degrees, i.e. first introducing an object from inlet path one and pushing the disinfected object out onto outlet path one, then rotating 45 degrees in a first direction to perform disinfection and thereafter further 45 degrees rotation in the first direction to get in line with path two for introducing a new object from inlet path two and deliver the lastly disinfected object onto the outlet path two. Then rotation is performed in an opposite (second) direction step wise as described above, thereafter again in the first direction, etc.

Further it is realized that the basic principle of the invention, e.g. to perform steam disinfection, may also work if both the inner disinfection chamber 31 and the outer cover 30 are arranged with merely one opening, e.g. and then first rotate a given angle/range (e.g. 45 degrees, or 45-90 degrees) to perform disinfection and after completed disinfection rotate back to have the object 5 with its handle in a position ready to grab/fetch. In such an application the inlet path and outlet path may be the same, e.g. no buffer path/s, but one object at the time is handled. Further it is evident that the expression "outer cover 30" must be given a broad interpretation, e.g. also including an embodiment where the outer cover 30 is in the form of an isolating layer applied directly onto the outer surface of the inner disinfection chamber 31.

Further it is realized that some of the features described above and/or defined in the claims may be used in combination with other devices, e.g. known prior art devices, and as consequence it is foreseen that some of such features may be subject for their own protection by means of filing divisional applications, e.g.:
- the basic principle of using one fixed part (e.g. the cover 30) and one rotatable part (e.g. the inner chamber 31) to achieve sealing of the inner chamber by means of rotation, e.g. to be able to effectively use steam as disinfectant.
- A disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is rotated by a drive unit (32) that is controlled by a control unit (6), wherein said inlet part (2) and said outlet part (4), respectively, are separated including a separate inlet path (20) and a separate outlet path (40), respectively,preferably providing space for a plurality of movable objects (5).
- A disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is rotated by a drive unit (32) that is controlled by a control unit (6), wherein said outer cover (30) and said inner disinfection chamber (31) are both rotatable, preferably by being attached to each other having a joint object opening (102, 310).
- A disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is rotated by a drive unit (32) that is controlled by a control unit (6), wherein said disinfectant supply arrangement (7) includes at least one supply nozzle (70), preferably a plurality of supply nozzles (70) and wherein said disinfectant supply arrangement (7) includes a disinfectant supply device (71), preferably including a steam generator (710), arranged to supply disinfectant, preferably steam, via a tubing (72) into said rotatable disinfection chamber (31).
- A disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is rotated by a drive unit (32) that is controlled by a control unit (6), wherein at least one of said inlet part (2) and said outlet part (4) includes a drive system (22, 23; 42, 43) including a first drive arrangement part (22; 42) and a second drive arrangement part (23; 43), preferably along both paths (2, 4), wherein more preferred said first drive arrangement part (22; 42) includes at least one pulling member (226A, 226B) protruding upwardly from a floor level and even more preferred wherein said second drive arrangement part (22; 42) includes at least one downwardly protruding gripping member (430) protruding downwardly from a level above the objects (5).
- A disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is rotated by a drive unit (32) that is controlled by a control unit (6), wherein said inlet part (2) includes a plurality of separate inlet paths (20) and said outlet part (4) includes a plurality separate outlet paths (40), and that one single disinfection unit (3) is arranged to disinfect moving objects (5) within a plurality, preferably all, of said plurality of paths (20, 40).
- A disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is rotated by a drive unit (32) that is controlled by a control unit (6), wherein said inlet part (2) and said outlet part (4) includes gate members (29, 49) at an inlet (21) and outlet (41), respectively, that may lock a movable object (5) to block the inlet and outlet passage, respectively.

## Claims

1. Disinfection arrangement for disinfecting a movable object (5), preferably adapted for shopping carts, comprising a disinfectant supply arrangement (7), an object inlet part (2), a disinfection unit (3) and an object outlet part (4), wherein said disinfection unit (3) comprises an outer cover (30) and an inner disinfection chamber (31), wherein one of said disinfection chamber (31) and/or outer cover (30) is is configured to rotate by a drive unit (32) that is controlled by a control unit (6), **characterized in that** said control unit (6) is arranged to control rotation such that after disinfection the movable object (5) is positioned in the outlet part (4) with the same side facing outwards as was facing outwards when the movable object was moved into the inlet part (2).

2. Disinfection arrangement according to claim 1, wherein said inlet part (2) and said outlet part (4), respectively, are separated including a separate inlet path (20) and a separate outlet path (40), respectively, preferably providing space for a plurality of movable objects (5).

3. Disinfection arrangement according to claim 1 or 2, wherein disinfection unit (3) comprises a bottom part (33) arranged to support said outer cover (30) and said inner disinfection chamber (31).

4. Disinfection arrangement according to claim 1, 2 or 3, wherein said outer cover (30) and said inner disinfection chamber (31) are both rotatable, preferably by being attached to each other having a joint object opening (102, 310).

5. Disinfection arrangement according to claim 4, wherein a door arrangement (34, 35) is arranged to close said joint opening (102, 310), preferably including an actuating member (35) enabling movement of a door member (34) between an open and closed position.

6. Disinfection arrangement according to claim 5, wherein a door member (34) of said door arrangement (34, 35) is arranged with disinfection supply device (347) connected to said disinfectant supply arrangement (7).

7. Disinfection arrangement according to any of claims 1- 3, wherein said outer cover (30) is fixed and said inner disinfection chamber (31) is rotatable, wherein preferably said outer cover (30) includes a larger number of openings (300, 302) than the number of opening/s (310) of the inner rotatable disinfection chamber (31), and more preferred said inner rotatable disinfection chamber (31) merely includes one object opening (310).

8. Disinfection arrangement according to 7, wherein said cover (30) includes non-permeable walls (301), preferably provided with rotation symmetric inner wall surface (304) enabling sealing of an object opening (310) of said disinfection chamber (31) against said inner walls surface (304) at least at one position, preferably also an angle range during rotation, to seal off the inner space within the disinfection chamber (31).

9. Disinfection arrangement according to any preceding claim, wherein said disinfectant supply arrangement (7) includes at least one supply nozzle (70), preferably a plurality of supply nozzles (70).

10. Disinfection arrangement according to claim 9 , wherein said disinfectant supply arrangement (7) includes a disinfectant supply device (71), preferably including a steam generator (710), arranged to supply disinfectant, preferably steam, via a tubing (72) into said rotatable disinfection chamber (31).

11. Disinfection arrangement according to any of claims 2-11, wherein at least one of said inlet part (2) and said outlet part (4) includes a drive system (22, 23; 42, 43) including a first drive arrangement part (22; 42) and a second drive arrangement part (23; 43), preferably along both paths (2, 4), wherein more preferred said first drive arrangement part (22; 42) includes at least one pulling member (226A, 226B) protruding upwardly from a floor level and even more preferred wherein said second drive arrangement part (22; 42) includes at least one downwardly protruding gripping member (430) protruding downwardly from a level above the objects (5).

12. Disinfection arrangement according to any of claims 2-11, wherein said inlet part (2) includes a plurality of separate inlet paths (20) and said outlet part (4) includes a plurality separate outlet paths (40), and that one single disinfection unit (3) is arranged to disinfect moving objects (5) within a plurality, preferably all, of said plurality of paths (20, 40).

13. Disinfection arrangement according to any preceding claim, wherein said inlet part (2) and said outlet part (4) includes gate members (29, 49) at an inlet (21) and outlet (41), respectively, that may lock a movable object (5) to block the inlet and outlet passage, respectively.

14. Method for disinfection of a movable object within a disinfection arrangement (1) according to claim 1, comprising the steps of:
- supplying at least one movable object (5) to an opening (21) of the object inlet part (2),
- moving said movable object (5) along the inlet path (20) of said object inlet part (2),
- introducing said movable object (5) into the disinfection unit (3), via an inlet opening (310) in said disinfection chamber (31),
- activating a drive unit (32) to rotate either the disinfection chamber (31) and/or the cover (30),
- activating the disinfectant supply arrangement (7), to perform disinfection of said movable object (5),
- continue rotation until said inlet opening (310) is in line with the outlet path (40),
- moving said movable object (5) out from the disinfection unit (3) and along the outlet path (40) of said object outlet part (4) to the outlet (41), such that after disinfection the movable object (5) is positioned in the outlet part (4) with the same side facing outwards as was facing outwards when the movable object was moved into the inlet part (2),
- introducing a new movable object (5) into the disinfection unit (3) and repeating said steps,
preferably including sealing said inlet opening (310) by interaction with a door arrangement (35) or the inner wall (304) of said cover (30),
more preferred providing said disinfection arrangement (1) with a plurality of inlet (20) and outlet paths (40), respectively and serving a plurality of inlet (20) and outlet paths (40), by one single disinfection unit (3).

15. Method according to claim 14, wherein said disinfection is achieved by use of steam as disinfectant.

## Patentansprüche

1. Desinfektionsanordnung zur Desinfektion eines beweglichen Gegenstands (5), vorzugsweise geeignet für Einkaufswagen, mit einer Desinfektionsmittelzuführungsanordnung (7), einem Gegenstandseinlassteil (2), einer Desinfektionseinheit (3) und einem Gegenstandsauslassteil (4), wobei die Desinfektionseinheit (3) eine äußere Abdeckung (30) und eine innere Desinfektionskammer (31) umfasst, wobei die Desinfektionskammer (31) und/oder die äußere Abdeckung (30) so ausgebildet ist, dass sie von einer Antriebseinheit (32) gedreht wird, die von einer Steuereinheit (6) gesteuert wird, **dadurch gekennzeichnet, dass** die Steuereinheit (6) dazu eingerichtet ist, die Drehung so zu steuern, dass der bewegliche Gegenstand (5) nach der Desinfektion mit der gleichen Seite nach außen gewandt in dem Auslassteil (4) positioniert ist, die nach außen gewandt war, als der bewegliche Gegenstand in den Einlassteil (2) befördert wurde.

2. Desinfektionsanordnung nach Anspruch 1, bei der das Einlassteil (2) und das Auslassteil (4) jeweils getrennt sind und eine separate Einlassbahn (20) bzw. eine separate Auslassbahn (40) aufweisen, die vorzugsweise Platz für mehrere bewegliche Gegenstände (5) bieten.

3. Desinfektionsanordnung nach Anspruch 1 oder 2, bei der die Desinfektionseinheit (3) ein Unterteil (33) umfasst, das so eingerichtet ist, dass es die äußere Abdeckung (30) und die innere Desinfektionskammer (31) trägt.

4. Desinfektionsanordnung nach Anspruch 1, 2 oder 3, bei der die äußere Abdeckung (30) und die innere Desinfektionskammer (31) beide drehbar sind, vorzugsweise indem sie aneinander befestigt sind und eine gemeinsame Gegenstandsöffnung (102, 310) aufweisen.

5. Desinfektionsanordnung nach Anspruch 4, bei der eine Türanordnung (34, 35) dazu eingerichtet ist, die gemeinsame Öffnung (102, 310) zu schließen, vorzugsweise mit einem Betätigungselement (35), das eine Bewegung eines Türelements (34) zwischen einer offenen und einer geschlossenen Stellung ermöglicht.

6. Desinfektionsanordnung nach Anspruch 5, bei der ein Türelement (34) der Türanordnung (34, 35) mit einer Desinfektionsmittelzuführungsvorrichtung (347) angeordnet ist, die mit der Desinfektionsmittelzuführungsanordnung (7) verbunden ist.

7. Desinfektionsanordnung nach einem der Ansprüche 1 - 3, bei der die äußere Abdeckung (30) feststehend und die innere Desinfektionskammer (31) drehbar ist, wobei vorzugsweise eine Anzahl an Öffnungen (300, 302) der äußeren Abdeckung (30) größer ist als die Anzahl an Öffnungen (310) der inneren drehbaren Desinfektionskammer (31) und bevorzugter die innere drehbare Desinfektionskammer (31) nur eine Gegenstandsöffnung (310) aufweist.

8. Desinfektionsanordnung nach Anspruch 7, bei der die Abdeckung (30) undurchlässige Wände (301) aufweist, die vorzugsweise mit einer rotationssymmetrischen Innenwandfläche (304) versehen sind, die ein Verschließen einer Gegenstandsöffnung (310) der Desinfektionskammer (31) gegenüber der Innenwandfläche (304) an wenigstens einer Position, vorzugsweise auch in einem Winkelbereich während der Drehung ermöglicht, um den Innenraum in der Desinfektionskammer (31) dicht zu verschließen.

9. Desinfektionsanordnung nach einem vorhergehenden Anspruch, bei der die Desinfektionsmittelzuführungsanordnung (7) wenigstens eine Zuführungsdüse (70), vorzugsweise mehrere Zuführungsdüsen (70), aufweist.

10. Desinfektionsanordnung nach Anspruch 9, bei der die Desinfektionsmittelzuführungsanordnung (7) eine Desinfektionsmittelzuführungsvorrichtung (71) aufweist, die vorzugsweise einen Dampferzeuger (710) aufweist, der dazu eingerichtet ist, Desinfektionsmittel, vorzugsweise Dampf, über eine Rohrleitung (72) in die drehbare Desinfektionskammer (31) zuzuführen.

11. Desinfektionsanordnung nach einem der Ansprüche 2 - 11, bei der der Einlassteil (2) und/oder der Auslassteil (4) ein Antriebssystem (22, 23; 42, 43) aufweisen/aufweist, das einen ersten Antriebsanordnungsteil (22; 42) und einen zweiten Antriebsanordnungsteil (23; 43) aufweist, vorzugsweise entlang beider Bahnen (2, 4), wobei bevorzugter der erste Antriebsanordnungsteil (22; 42) mindestens ein Zugelement (226A, 226B) aufweist, das von einer Bodenebene nach oben ragt, und noch bevorzugter wobei der zweite Antriebsanordnungsteil (22; 42) mindestens ein nach unten ragendes Greifelement (430) aufweist, das von einer Ebene oberhalb der Gegenstände (5) nach unten ragt.

12. Desinfektionsanordnung nach einem der Ansprüche 2 - 11, bei der der Einlassteil (2) mehrere separate Einlassbahnen (20) und der Auslassteil (4) mehrere separate Auslassbahnen (40) aufweist und dass eine einzige Desinfektionseinheit (3) so eingerichtet ist, dass sie bewegliche Gegenstände (5) innerhalb mehrerer, vorzugsweise aller, der mehreren Bahnen (20, 40) desinfiziert.

13. Desinfektionsanordnung nach einem vorhergehenden Anspruch, bei der der Einlassteil (2) und der Auslassteil (4) an einem Einlass (21) bzw. einem Auslass (41) Torelemente (29, 49) aufweisen, die einen beweglichen Gegenstand (5) so arretieren können, dass der Einlass- bzw. Auslassdurchgang blockiert ist.

14. Verfahren zur Desinfektion eines beweglichen Gegenstands in einer Desinfektionsanordnung (1) nach Anspruch 1, mit den folgenden Schritten:
- Liefern wenigstens eines beweglichen Gegenstands (5) zu einer Öffnung (21) des Gegenstandseinlassteils (2),
- Befördern des beweglichen Gegenstands (5) entlang der Einlassbahn (20) des Gegenstandseinlassteils (2),
- Einführen des beweglichen Gegenstands (5) in die Desinfektionseinheit (3) über eine Einlassöffnung (310) in der Desinfektionskammer (31),
- Aktivieren einer Antriebseinheit (32) zum Drehen entweder der Desinfektionskammer (31) und/oder der Abdeckung (30),
- Aktivieren der Desinfektionsmittelzuführungsanordnung (7) zur Durchführung einer Desinfektion des beweglichen Gegenstands (5),
- Fortsetzen der Drehung, bis die Einlassöffnung (310) auf die Auslassbahn (40) ausgerichtet ist,
- Befördern des beweglichen Gegenstands (5) aus der Desinfektionseinheit (3) heraus und entlang der Auslassbahn (40) des Gegenstandsauslassteils (4) zum Auslass (41), derart, dass der bewegliche Gegenstand (5) nach der Desinfektion mit der gleichen Seite nach außen gewandt in dem Auslassteil (4) positioniert ist, die nach außen gewandt war, als der bewegliche Gegenstand in den Einlassteil (2) befördert wurde,
- Einführen eines neuen beweglichen Gegenstands (5) in die Desinfektionseinheit (3) und Wiederholen der Schritte,
wobei vorzugsweise dabei die Einlassöffnung (310) durch Zusammenwirken mit einer Türanordnung (35) oder der Innenwand (304) der Abdeckung (30) verschlossen wird,
bevorzugter wobei die Desinfektionsanordnung (1) mit mehreren Einlass- (20) bzw. Auslassbahnen (40) bereitgestellt wird und mehrere Einlass- (20) und Auslassbahnen (40) von einer einzigen Desinfektionseinheit (3) bedient werden.

15. Verfahren nach Anspruch 14, bei dem die Desinfektion durch Verwendung von Dampf als Desinfektionsmittel erzielt wird.

## Revendications

1. Agencement de désinfection destiné à désinfecter un objet mobile (5), de préférence adapté pour des caddies, comprenant un agencement d'alimentation en désinfectant (7), une partie d'entrée (2) d'objet, une unité de désinfection (3) et une partie de sortie (4) d'objet, dans lequel ladite unité de désinfection (3) comprend un capot (30) externe et une chambre de désinfection (31) interne, dans lequel l'un de ladite chambre de désinfection (31) et/ou dudit capot (30) externe est configuré pour être mis en rotation par une unité d'entraînement (32) qui est commandée par une unité de commande (6), **caractérisé en ce que** ladite unité de commande (6) est agencée pour commander la rotation de telle sorte qu'après la désinfection, l'objet mobile (5) est positionné dans la partie de sortie (4) avec le même côté tourné vers l'extérieur qui était tourné vers l'extérieur lorsque l'objet mobile a été déplacé dans la partie d'entrée (2).

2. Agencement de désinfection selon la revendication 1, dans lequel ladite partie d'entrée (2) et ladite partie de sortie (4), respectivement, sont séparées, comportant un trajet d'entrée (20) séparé et un trajet de sortie (40) séparé, respectivement, fournissant de préférence un espace pour une pluralité d'objets mobiles (5).

3. Agencement de désinfection selon la revendication 1 ou 2, dans lequel l'unité de désinfection (3) comprend une partie fond (33) agencée pour supporter ledit capot (30) externe et ladite chambre de désinfection (31) interne.

4. Dispositif de désinfection selon la revendication 1, 2 ou 3, dans lequel ledit capot (30) externe et ladite chambre de désinfection (31) interne sont tous deux rotatifs, de préférence en étant fixés l'un à l'autre avec une ouverture (102, 310) d'objet commune.

5. Agencement de désinfection selon la revendication 4, dans lequel un agencement de trappe (34, 35) est agencé pour fermer ladite ouverture (102, 310) commune, comportant de préférence un élément d'actionnement (35) permettant le déplacement d'un élément trappe (34) entre une position ouverte et une position fermée.

6. Agencement de désinfection selon la revendication 5, dans lequel un élément trappe (34) dudit agencement de trappe (34, 35) est agencé avec un dispositif d'alimentation de désinfection (347) relié audit dispositif d'alimentation en désinfectant (7).

7. Agencement de désinfection selon l'une quelconque des revendications 1 à 3, dans lequel ledit capot (30) externe est fixe et ladite chambre de désinfection (31) interne est rotative, dans lequel de préférence ledit capot (30) externe comporte un nombre d'ouvertures (300, 302) supérieur au nombre d'ouvertures (310) de la chambre de désinfection (31) interne rotative, et plus préférablement ladite chambre de désinfection (31) interne rotative comporte seulement une ouverture (310) d'objet.

8. Agencement de désinfection selon la revendication 7, dans lequel ledit capot (30) comporte des parois (301) non perméables, de préférence pourvues d'une surface de paroi interne (304) symétrique en rotation permettant de sceller une ouverture (310) d'objet de ladite chambre de désinfection (31) contre ladite surface de paroi interne (304) au moins à une position, de préférence également une plage angulaire pendant la rotation, pour sceller l'espace interne au sein de la chambre de désinfection (31).

9. Agencement de désinfection selon l'une quelconque des revendications précédentes, dans lequel ledit dispositif d'alimentation en désinfectant (7) comporte au moins une buse d'alimentation (70), de préférence une pluralité de buses d'alimentation (70).

10. Agencement de désinfection selon la revendication 9, dans lequel ledit agencement d'alimentation en désinfectant (7) comporte un dispositif d'alimentation en désinfectant (71), comportant de préférence un générateur de vapeur (710), agencé pour alimenter en désinfectant, de préférence de la vapeur, par l'intermédiaire d'une tubulure (72) ladite chambre de désinfection (31) rotative.

11. Agencement de désinfection selon l'une quelconque des revendications 2 à 11, dans lequel au moins l'une de ladite partie d'entrée (2) et de ladite partie de sortie (4) comporte un système d'entraînement (22, 23 ; 42, 43) comportant une première partie d'agencement d'entraînement (22 ; 42) et une deuxième partie d'agencement d'entraînement (23 ; 43), de préférence le long des deux trajets (2, 4), dans lequel plus préférablement ladite première partie d'agencement d'entraînement (22 ; 42) comporte au moins un élément de traction (226A, 226B) faisant saillie vers le haut à partir d'un niveau de plancher et encore plus préférablement dans lequel ladite deuxième partie d'agencement d'entraînement (22 ; 42) comporte au moins un élément de préhension (430) faisant saillie vers le bas à partir d'un niveau situé au-dessus des objets (5).

12. Agencement de désinfection selon l'une quelconque des revendications 2 à 11, dans lequel ladite partie d'entrée (2) comporte une pluralité de trajets d'entrée (20) séparés et ladite partie de sortie (4) comporte une pluralité de trajets de sortie (40) séparés, et une seule unité de désinfection (3) est agencée pour désinfecter des objets (5) mobiles au sein d'une pluralité, de préférence l'ensemble, de ladite pluralité de trajets (20, 40).

13. Agencement de désinfection selon l'une quelconque des revendications précédentes, dans lequel ladite partie d'entrée (2) et ladite partie de sortie (4) comportant des éléments formant porte (29, 49) au niveau d'une entrée (21) et d'une sortie (41), respectivement, qui peuvent bloquer un objet (5) mobile pour bloquer le passage d'entrée et de sortie, respectivement.

14. Procédé de désinfection d'un objet mobile au sein d'un agencement de désinfection (1) selon la revendication 1, comprenant les étapes consistant à :
- fournir au moins un objet (5) mobile à une ouverture (21) de la partie d'entrée (2) d'objet,
- déplacer ledit objet (5) mobile le long du trajet d'entrée (20) de ladite partie d'entrée (2) d'objet, - introduire ledit objet (5) mobile dans l'unité de désinfection (3), par l'intermédiaire d'une ouverture (310) d'entrée dans ladite chambre de désinfection (31),
- activer une unité d'entraînement (32) pour mettre en rotation la chambre de désinfection (31) et/ou le capot (30),
- activer l'agencement d'alimentation en désinfectant (7), pour réaliser la désinfection dudit objet (5) mobile,
- poursuivre la rotation jusqu'à ce que ladite ouverture (310) d'entrée soit alignée avec le trajet de sortie (40), - déplacer ledit objet (5) mobile hors de l'unité de désinfection (3) et le long du trajet de sortie (40) de ladite partie de sortie (4) d'objet jusqu'à la sortie (41), de sorte qu'après la désinfection, l'objet (5) mobile est positionné dans la partie de sortie (4) avec le même côté tourné vers l'extérieur qui était tourné vers l'extérieur lorsque l'objet mobile a été déplacé dans la partie d'entrée (2),
- introduire un nouvel objet (5) mobile dans l'unité de désinfection (3) et répéter lesdites étapes,
comportant de préférence l'étanchéification de ladite ouverture (310) d'entrée par l'interaction avec un agencement de trappe (35) ou la paroi interne (304) dudit capot (30), plus préférablement la fourniture audit agencement de désinfection (1) d'une pluralité de trajets d'entrée (20) et de sortie (40), respectivement, et la desserte d'une pluralité de trajets d'entrée (20) et de sortie (40), par une seule unité de désinfection (3).

15. Procédé selon la revendication 14, dans lequel ladite désinfection est réalisée par l'utilisation de vapeur en tant que désinfectant.
